(19) Europäisches Patentamt / European Patent Office / Office européen des brevets

(11) **EP 3 750 923 A1**

(12) **EUROPEAN PATENT APPLICATION**
published in accordance with Art. 153(4) EPC

(43) Date of publication:
**16.12.2020 Bulletin 2020/51**

(21) Application number: **19748215.1**

(22) Date of filing: **02.02.2019**

(51) Int Cl.:
*C08B 37/00* (2006.01)          *A61K 31/715* (2006.01)
*A61P 3/06* (2006.01)           *A61P 3/10* (2006.01)

(86) International application number:
**PCT/CN2019/074601**

(87) International publication number:
**WO 2019/149284 (08.08.2019 Gazette 2019/32)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **05.02.2018 CN 201810114240**

(71) Applicants:
• **Shanghai Green Valley Pharmaceutical Co., Ltd.**
  **Pudong New Area, Shanghai 201203 (CN)**
• **Soochow University**
  **Suzhou, Jiangsu 215213 (CN)**

(72) Inventors:
• **ZHANG, Zhenqing**
  **Shanghai 201203 (CN)**
• **XU, Naiyu**
  **Suzhou, Jiangsu 215123 (CN)**
• **YIN, Xiang**
  **Shanghai 201203 (CN)**
• **PANG, Li**
  **Shanghai 201203 (CN)**
• **SHEN, Lulu**
  **Shanghai 201203 (CN)**
• **XUE, Jie**
  **Suzhou, Jiangsu 215123 (CN)**

(74) Representative: **Plasseraud IP**
  **66, rue de la Chaussée d'Antin**
  **75440 Paris Cedex 09 (FR)**

(54) **SEPARATED SAPOSHNIKOVIA DIVARICATA POLYSACCHARIDE AND USE THEREOF**

(57) The present application relates to the field of medicine. The present application relates to an isolated *Saposhnikovia divaricata* polysaccharide and use thereof in the manufacture of a medicament for treating diabetes mellitus or hyperlipoidemia. In particular, the present application relates to an isolated *Saposhnikovia divaricata* polysaccharide comprising *L*-arabinose, *D*-galacturonic acid, *D*-mannose, *D*-glucose and *D*-galactose, wherein the molar ratio of the *L*-arabinose: *D*-galacturonic acid: *D*-mannose: *D*-glucose: *D*-galactose is 1-15 : 1-10 : 1-10 : 10-40 : 1-15, preferably 1-5 : 5-10: 1-5 : 20-25 : 1-5.

EP 3 750 923 A1

**Description**

**TECHNICAL FIELD**

The present application relates to the field of medicine. Specifically, the present application relates to an isolated *Saposhnikovia divaricata* polysaccharide (SDP) and use thereof in the manufacture of a medicament for treating diabetes mellitus or hyperlipoidemia.

**BACKGROUND OF THE INVENTION**

Researches in recent years have found that carbohydrates are not only a type of important structural and energy substances, but also have important biological functions. Carbohydrates are involved in the processes of mutual recognition and information transmission between cells, and are considered as another type of important informational molecules besides nucleic acids in organisms. Moreover, carbohydrates are also key factors for cell surface signal recognition, antigen-antibody reactions, and information transmission and perception between cells. Therefore, researches on polysaccharides with biological activities attract increasing attention. Due to the complex structures of carbohydrates, their separation and structural identification are difficult. So far, only coriolus versicolor polysaccharide, polyporus polysaccharide, lentinan, schizophyllan, pachymaran and the like have been used clinically. There is a need for more bioactive polysaccharides in the art.

The traditional Chinese medicinal material *Saposhnikovia divaricata* refers to the dried root of the plant before development of pedicel of *Saposhnikovia divaricata,* an *Umbelliferae* plant. *Saposhnikovia divaricata* is often used to treat diseases such as cold, headache, rheumatism, paralysis, rubella, pruritus, and tetanus.

The main chemical components of *Saposhnikovia divaricata* include volatile oils, chromone, coumarin, organic acids, *Saposhnikovia divaricata* polysaccharides, etc. *Saposhnikovia divaricata* polysaccharide is a branched polysaccharide formed by connection of multiple monosaccharides. Generally, *Saposhnikovia divaricata* polysaccharides are characterized by the composition of the monosaccharides contained therein and the mode of connection thereof. The *Saposhnikovia divaricata* polysaccharides prepared by different extraction methods have different monosaccharide compositions and connection modes. Hongxia Dou, et al. (Research progress on the chemical components and pharmacological effects of Saposhnikovia divaricata, Information on Traditional Chinese Medicine, 2009, 26(2), 15) extracted a variety of *Saposhnikovia divaricata* polysaccharides from *Saposhnikovia divaricata*: XC-1 (with an average molecular weight of 13100), XC-2 (with an average molecular weight of 73500), and Saponikovan A, B, and C (with molecular weights of 54000, 280000, and 132000, respectively). *Saposhnikovia divaricata* polysaccharides are generally used in the art for anti-tumor, anti-oxidation, and improvement of immunity of the body. There is no report about use of *Saposhnikovia divaricata* polysaccharide for treatment of diabetes mellitus or hyperlipoidemia till now.

Diabetes mellitus refers to a metabolic disease characterized by high blood glucose due to insulin secretion dysfunction and/or the inability of insulin to exert normal physiological effects. Diabetes mellitus can also cause a variety of complications, such as diabetic heart diseases, diabetic eye diseases, and diabetic vascular diseases. Diabetes mellitus is a chronic disease that seriously threatens public health.

Hyperlipoidemia refers to a metabolic disease in which the levels of one or more lipids in the blood are abnormal (for example, multiple lipids are present in a level higher than the normal level). Hyperlipoidemia is manifested as too high levels of total cholesterol (TC), triglyceride (TG) and low-density lipoprotein cholesterol (LDL-C) or a too low level of high-density lipoprotein cholesterol (HDL-C) in the blood. In recent years, the incidence of hyperlipoidemia increasingly grows. Hyperlipoidemia is also closely associated with some severe cardiovascular and cerebrovascular diseases such as atherosclerosis and coronary heart disease.

**SUMMARY OF THE INVENTION**

Due to the complex structures of carbohydrates, different extraction processes will directly affect the structural composition of polysaccharides, thereby affecting their efficacies. The present invention provides an improved method for preparing *Saposhnikovia divaricata* polysaccharide, comprising a step of gradient precipitation. It was found from structural analysis that the isolated *Saposhnikovia divaricata* polysaccharide of the present invention had a completely different structure from the known *Saposhnikovia divaricata* polysaccharides. It was verified by animal experiments that the isolated *Saposhnikovia divaricata* polysaccharide of the present invention had potential effects of treating diabetes mellitus and regulating blood lipids.

In one aspect, the present application provides an isolated *Saposhnikovia divaricata* polysaccharide, comprising monosaccharides such as *L*-arabinose (*L*-Ara), *D*-galacturonic acid (*D*-GlaA), *D*-mannose (*D*-Man), *D*-glucose (*D*-Glc) and *D*-galactose (*D*-Gal), wherein the molar ratio of the *L*-arabinose: *D*-galacturonic acid: *D*-mannose: *D*-glucose: *D*-galactose is 1-15 : 1-10 : 1-10 : 10-40 : 1-15. In one embodiment, the molar ratio of the *L*-arabinose: *D*-galacturonic acid:

*D*-mannose: *D*-glucose: *D*-galactose is 1-5 : 5-10 : 1-5 : 20-25 : 1-5.

In one embodiment, the monosaccharide components contained in the isolated *Saposhnikovia divaricata* polysaccharide are connected to each other in a specific manner. The *L*-arabinose includes 1,4-linked *L*-arabinose and/or 1,3,4-linked *L*-arabinose; the *D*-galacturonic acid includes terminal D-galacturonic acid; the *D*-mannose includes 1,6-linked *D*-mannose; the *D*-glucose includes 1,4-linked *D*-glucose and/or 1,3,6-linked *D*-glucose; and the *D*-galactose includes terminal *D*-galactose and/or 1,4-linked *D*-galactose.

In one preferred embodiment, the *L*-arabinose as described in the present application includes 1,4-linked *L*-arabinose and/or 1,3,4-linked *L*-arabinose.

In one preferred embodiment, the *D*-galacturonic acid as described in the present application includes terminal *D*-galacturonic acid.

In one preferred embodiment, the *D*-mannose as described in the present application includes 1,6-linked *D*-mannose.

In one preferred embodiment, the *D*-glucose as described in the present application includes 1,4-linked *D*-glucose and/or 1,3,6-linked *D*-glucose.

In one preferred embodiment, the *D*-galactose as described in the present application includes terminal *D*-galactose and/or 1,4-linked *D*-galactose.

In one embodiment, the isolated *Saposhnikovia divaricata* polysaccharide comprises 1,4-linked *L*-arabinose, 1,3,4-linked *L*-arabinose, terminal *D*-galacturonic acid, 1,6-linked *D*-mannose, 1,4-linked *D*-glucose, 1,3,6-linked *D*-glucose, terminal *D*-galactose, and 1,4-linked *D*-galactose. In another preferred embodiment, the molar ratio of the 1,4-linked *L*-arabinose: 1,3,4-linked *L*-arabinose: terminal *D*-galacturonic acid: 1,6-linked *D*-mannose: 1,4-linked *D*-glucose: 1,3,6-linked *D*-glucose: terminal *D*-galactose: 1,4-linked *D*-galactose is 1-5 : 1-10 : 1-10 : 1-10 : 10-30 : 1-10 : 1-5 : 1-10, preferably 1-3 : 1-5 : 5-10 : 1-5 : 15-25 : 1-5 : 1-3 : 1-5.

In one preferred embodiment, the present application provides an isolated *Saposhnikovia divaricata* polysaccharide, comprising *L*-arabinose, *D*-galacturonic acid, *D*-mannose, *D*-glucose and *D*-galactose, wherein the *L*-arabinose includes 1,4-linked *L*-arabinose and/or 1,3,4-linked *L*-arabinose; the *D*-galacturonic acid includes terminal *D*-galacturonic acid; the *D*-mannose includes 1,6-linked *D*-mannose; the *D*-glucose includes 1,4-linked *D*-glucose and/or 1,3,6-linked *D*-glucose; and the *D*-galactose includes terminal *D*-galactose and/or 1,4-linked *D*-galactose. In a further preferred embodiment, the molar ratio of the 1,4-linked *L*-arabinose: 1,3,4-linked *L*-arabinose: terminal *D*-galacturonic acid: 1,6-linked *D*-mannose: 1,4-linked *D*-glucose: 1,3,6-linked *D*-glucose: terminal *D*-galactose: 1,4-linked *D*-galactose is 1-5 : 1-10 : 1-10 : 1-10 : 10-30 : 1-10 : 1-5 : 1-10, preferably 1-3 : 1-5 : 5-10 : 1-5 : 15-25 : 1-5 : 1-3 : 1-5.

In another embodiment, one or more of the monosaccharide components are pyranose. In one preferred embodiment, the monosaccharide components are all pyranose.

In one preferred embodiment, the isolated *Saposhnikovia divaricata* polysaccharide as described in the present application has a molecular weight ranging from $5 \times 10^4$ to $5 \times 10^5$ Da, preferably $1 \times 10^5$ to $3.5 \times 10^5$ Da.

In another aspect, the present application provides a method for preparing the isolated *Saposhnikovia divaricata* polysaccharide, comprises the steps of:

(1) extracting *Saposhnikovia divaricata* with water one or more times to give an aqueous extract of *Saposhnikovia divaricata,* optionally concentrating the aqueous extract of *Saposhnikovia divaricata*;

(2) adding an organic solvent to the optionally concentrated aqueous extract of *Saposhnikovia divaricata* to give an mixture containing the organic solvent at a concentration of 15-30%, centrifuging the mixture to give a supernatant;

(3) adding an organic solvent to the supernatant to give a mixture containing the organic solvent at a concentration of 70-90%, centrifuging the mixture to give a precipitate; and

(4) drying the precipitate to obtain the isolated *Saposhnikovia divaricata* polysaccharide.

In one embodiment, the concentration of the organic solvent in step (2) is preferably 17-28%, more preferably 20-25%. In some embodiments, step (2) is also referred to as the first gradient precipitation.

In one embodiment, the concentration of the organic solvent in step (3) is preferably 75-85%, more preferably 80-85%.

In one embodiment, the volume : weight ratio of water to the *Saposhnikovia divaricata* in step (1) is 8:1 to 30:1, preferably 20:1 to 30:1.

In one embodiment, the extraction temperature in step (1) is 40-100 °C, preferably 60-100 °C, more preferably 80-100 °C, and most preferably 90-95 °C.

In one embodiment, the extraction time in step (1) is 1-4 hours, preferably 1-2 hours.

In one embodiment, in step (1), the *Saposhnikovia divaricata* is extracted with water one or more times.

In another preferred embodiment, in step (1), the *Saposhnikovia divaricata* is extracted with water 1, 2, 3, or 4 times.

In one embodiment, the method further comprises step (3') between steps (3) and (4): dissolving the precipitate obtained from step (3) with water to give an aqueous solution, adding an organic solvent to the aqueous solution to give a mixture containing the organic solvent at a concentration of 70-90%, preferably 75-85%, and more preferably 80-85%, and centrifuging the mixture to give a precipitate; wherein step (3') can be repeated one or more times, preferably 1, 2 or 3 times.

In some embodiments, steps (3) and/or (3') are also referred to as the second gradient precipitation.

In one embodiment, the organic solvent in step (2) and/or (3) and/or (3') is selected from methanol, ethanol, propanol, acetone, or a mixture thereof, preferably ethanol.

The *Saposhnikovia divaricata* as described in the present application include the commercially available medicinal material *Saposhnikovia divaricata* (i.e., the dried root of the plant before development of pedicel of *Saposhnikovia divaricata*) and decoction pieces of *Saposhnikovia divaricata*. In one embodiment, the *Saposhnikovia divaricata* as described in the present application is a decoction piece of *Saposhnikovia divaricata*.

The term "isolated *Saposhnikovia divaricata* polysaccharide" refers to the *Saposhnikovia divaricata* polysaccharide isolated from the natural state where its original plant raw material exists naturally by an artificial means (such as extraction, purification, or the like). The plant raw material can be *Saposhnikovia divaricata* in the form of a plant or *Saposhnikovia divaricatal* in the form of a medicinal material, such as the dried root of the plant before development of pedicel of *Saposhnikovia divaricata* or decoction pieces of *Saposhnikovia divaricata*.

In another aspect, the present application provides an isolated *Saposhnikovia divaricata* polysaccharide, which is obtained by the method of preparation as described in the present application. In one preferred embodiment, the isolated *Saposhnikovia divaricata* polysaccharide comprises *L*-arabinose, *D*-galacturonic acid, *D*-mannose, *D*-glucose and *D*-galactose, wherein the molar ratio of the *L*-arabinose: *D*-galacturonic acid: *D*-mannose: *D*-glucose: *D*-galactose is 1-15 : 1-10 : 1-10 : 10-40 : 1-15, preferably 1-5 : 5-10 : 1-5 : 20-25 : 1-5. In one preferred embodiment, the *L*-arabinose includes 1,4-linked *L*-arabinose and/or 1,3,4-linked *L*-arabinose; the *D*-galacturonic acid includes terminal *D*-galacturonic acid; the *D*-mannose includes 1,6-linked *D*-mannose; the *D*-glucose includes 1,4-linked *D*-glucose and/or 1,3,6-linked *D*-glucose; and the *D*-galactose includes terminal *D*-galactose and/or 1,4-linked *D*-galactose. In a further preferred embodiment, the molar ratio of the 1,4-linked *L*-arabinose: 1,3,4-linked *L*-arabinose: terminal *D*-galacturonic acid: 1,6-linked *D*-mannose: 1,4-linked *D*-glucose: 1,3,6-linked *D*-glucose: terminal *D*-galactose: 1,4-linked *D*-galactose is 1-5 : 1-10 : 1-10 : 1-10 : 10-30 : 1-10 : 1-5 : 1-10, preferably 1-3 : 1-5 : 5-10 : 1-5 : 15-25 : 1-5 : 1-3 : 1-5.

The 1,4-linked *L*-arabinose refers to *L*-arabinose connected to adjacent groups (e.g., adjacent monosaccharide residues) through glycosidic bonds at positions 1 and 4 of the sugar ring.

The 1,3,4-linked *L*-arabinose refers to *L*-arabinose connected to adjacent groups (e.g., adjacent monosaccharide residues) through glycosidic bonds at positions 1, 3 and 4 of the sugar ring.

The terminal *D*-galacturonic acid refers to *D*-galacturonic acid connected to an adjacent group (e.g., an adjacent monosaccharide residue) through a glycosidic bond at position 1 of the sugar ring.

The 1,6-linked *D*-mannose refers to *D*-mannose connected to adjacent groups (e.g., adjacent monosaccharide residues) through glycosidic bonds at positions 1 and 6 of the sugar ring.

The 1,4-linked *D*-glucose refers to *D*-glucose connected to adjacent groups (e.g., adjacent monosaccharide residues) through glycosidic bonds at positions 1 and 4 of the sugar ring.

The 1,3,6-linked *D*-glucose refers to *D*-glucose connected to adjacent groups (e.g., adjacent monosaccharide residues) through glycosidic bonds at positions 1, 3 and 6 of the sugar ring.

The terminal *D*-galactose refers to *D*-galactose connected to an adjacent group (e.g., an adjacent monosaccharide residue) through a glycosidic bond at position 1 of the sugar ring.

The 1,4-linked *D*-galactose refers to *D*-galactose connected to adjacent groups (e.g., adjacent monosaccharide residues) through glycosidic bonds at positions 1 and 4 of the sugar ring.

The sugar as described in the present application can be in $\alpha$-configuration or $\beta$-configuration.

In another aspect, the present application provides use of the isolated *Saposhnikovia divaricata* polysaccharide obtained in the present invention in the manufacture of a medicament for treating diabetes mellitus or hyperlipoidemia.

In another aspect, the present application provides a pharmaceutical composition, comprising a therapeutically effective amount of the isolated *Saposhnikovia divaricata* polysaccharide obtained in the present invention, and a pharmaceutically acceptable carrier.

In one preferred embodiment, the pharmaceutical composition is a tablet, capsule, granule, syrup, suspension, solution, dispersion, sustained-release formulation for oral or non-oral administration, formulation for intravenous injection, formulation for subcutaneous injection, inhalation formulation, transdermal formulation, rectal or vaginal suppository.

The pharmaceutically acceptable carrier as described in the present application refers to a pharmaceutically acceptable carrier well known to those skilled in the art. The pharmaceutically acceptable carriers in the present application include, but are not limited to, fillers, wetting agents, binders, disintegrants, lubricants, adhesives, glidants, taste-masking agents, surfactants, preservatives, etc. Fillers include, but are not limited to, lactose, microcrystalline cellulose, starch, powdered sugar, dextrin, mannitol, calcium sulfate, etc. Wetting agents and binders include, but are not limited to, sodium carboxymethyl cellulose, hydroxypropyl cellulose, hydroxypropyl methyl cellulose, gelatin, sucrose, polyvinylpyrrolidone, etc. Disintegrants include, but are not limited to, sodium starch glycolate, cross-linked polyvinylpyrrolidone, cross-linked sodium carboxymethyl cellulose, low-substituted hydroxypropyl cellulose, etc. Lubricants include, but are not limited to, magnesium stearate, micronized silica gel, talc, hydrogenated vegetable oil, polyethylene glycol, magnesium lauryl sulfate, etc. Adhesives include, but are not limited to, arabic gum, alginic acid, calcium carboxymethyl cellulose, sodium carboxymethyl cellulose, dextrates, dextrin, dextrose, ethyl cellulose, gelatin, liquid glucose, guar gum, hydroxyethyl cellulose, hydroxypropyl cellulose, hydroxypropyl methyl cellulose, magnesium aluminum silicate, maltodextrin, methyl cellulose, polymethacrylate, polyvinylpyrrolidone, pre-gelatinized starch, sodium alginate, sorbitol, starch, syrup and tragacanth. Glidants include, but are not limited to, colloidal silicon dioxide, powdered cellulose, magnesium trisilicate, silicon dioxide, and talc. Taste-masking agents include, but are not limited to, aspartame, stevioside, fructose, glucose, syrup, honey, xylitol, mannitol, lactose, sorbitol, maltitol, and glycyrrhizin. Surfactants include, but are not limited to, Tween-80, and poloxamer. Preservatives include, but are not limited to, paraben, sodium benzoate, potassium sorbate, etc.

Methods for preparing various pharmaceutical compositions comprising active ingredients in various ratios are known in the art or are obvious to those skilled in the art according to the disclosure of the present application, as described in REMINGTON'S PHARMACEUTICAL SCIENCES, Martin, E.W., ed., Mack Publishing Company, 19th ed. (1995). The method for preparing the pharmaceutical composition comprises incorporating a suitable pharmaceutical excipient, carrier, diluent, or the like. The pharmaceutical composition described in the present application is prepared by a known method, including conventional mixing, dissolving or freeze-drying processes.

In the pharmaceutical composition described in the present application, the proportion of the active ingredient can vary from about 0.01% to about 99% of the weight of a given unit dosage form. In such therapeutically useful pharmaceutical composition formulations, the amount of the active ingredient is such that an effective dosage level can be achieved.

The tablet, capsule or the like as described in the present application can comprise: a binder, such as tragacanth, arabic gum, corn starch, or gelatin; an excipient, such as dicalcium hydrogen phosphate; a disintegrant, such as corn starch, potato starch, alginic acid, or the like; a lubricant, such as magnesium stearate; a sweetener, such as sucrose, fructose, lactose, or aspartame; or a flavoring agent, such as peppermint, wintergreen oil, or cherry flavor. When the unit dosage form is a capsule, in addition to the above types of materials, it can comprise a liquid carrier such as vegetable oil or polyethylene glycol. Various additional materials may be present as a coating or otherwise modify the physical form of the solid unit dosage form. For example, the tablet or capsule can be coated with gelatin, wax, shellac, sugar, or the like. The syrup can comprise an active ingredient, sucrose or fructose as a sweetener, methyl or propyl paraben as a preservative, a dye and a flavoring agent (such as cherry flavor or orange flavor). Of course, any material used for preparing any unit dosage form should be pharmaceutically acceptable and non-toxic in the amount used. In addition, the active ingredient can be incorporated into a sustained-release formulation and a sustained-release device.

The active ingredient can also be administered intravenously or intraperitoneally by infusion or injection. A solution of the active ingredient or a salt thereof in water can be prepared, and optionally mixed with a non-toxic surfactant. A dispersion in glycerin, liquid polyethylene glycol, glyceryl triacetate, or a mixture thereof, or an oil can also be prepared. Such formulations comprise a preservative to prevent the growth of microorganisms under ordinary conditions of storage and use.

The dosage form of the pharmaceutical composition suitable for injection or infusion can include a sterile aqueous solution, dispersion or sterile powder comprising an active ingredient (optionally, encapsulated in a liposome) suitable for an immediate formulation of a sterile injectable or infusible solution or dispersion. In all cases, the final dosage form must be sterile, liquid and stable under the conditions of production and storage. The liquid carrier can be a solvent or a liquid dispersion medium, including, for example, water, ethanol, polyol (such as, glycerol, propylene glycol, liquid polyethylene glycol, or the like), vegetable oil, non-toxic glyceride, or a suitable mixture thereof. The proper fluidity can be maintained, for example, by formation of liposomes, by maintaining the desired particle size in the case of a dispersion, or by use of a surfactant. Various antibacterial and antifungal agents (such as parabens, chlorobutanol, phenol, sorbic acid, thimerosal, etc.) can be used to prevent microorganisms. In many cases, it is preferable to include an isotonic agent such as a sugar, buffer or sodium chloride. Prolonged absorption of an injectable composition can be produced by using a composition that delays absorption (for example, aluminum monostearate and gelatin).

A sterile injectable solution is prepared by combining the required amount of the active ingredient in a suitable solvent with various additional ingredients listed above as required, followed by sterile filtration. In the case of sterile powders for preparing a sterile solution for injection, preferred methods of preparation are vacuum drying and freeze-drying techniques, which produce a powder of the active ingredient plus any other required components present in the sterile filtered solution.

Useful solid carriers include pulverized solids (such as talc, clay, microcrystalline cellulose, silica, alumina, etc.). Useful liquid carriers include water, ethanol or ethylene glycol, or a water-ethanol/ethylene glycol mixture, in which the pharmaceutical composition of the present application can be dissolved or dispersed in an effective amount optionally with the help of a non-toxic surfactant. An adjuvant (such as fragrance) and an additional antimicrobial agent can be added to optimize the property for a given use.

A thickener (such as a synthetic polymer, fatty acid, fatty acid salt and ester, fatty alcohol, modified cellulose or modified inorganic material) can also be used with a liquid carrier to form a coatable paste, gel, ointment, soap, or the like, which can be directly applied to the user's skin.

The therapeutically effective amount of the active ingredient not only depends on the specific salt selected, but also depends on the mode of administration, the nature of the disease to be treated, and the age and status of the patient, and ultimately depends on the decision of the attending physician or clinician.

The above formulations can be presented in a unit dosage form, which is physically discrete units containing a unit dose, and is suitable for administration to human and other mammals. The unit dosage form can be a capsule or a tablet. Depending on the specific treatment involved, the unit dose of the active ingredient can vary from or be adjusted between about 0.01 to about 1000 mg or more.

In another aspect, the present application provides use of a pharmaceutical composition comprising a therapeutically effective amount of the isolated *Saposhnikovia divaricata* polysaccharide obtained by the present invention in the manufacture of a medicament for treating diabetes mellitus or hyperlipoidemia.

In yet another aspect, the present application provides a method for treating diabetes mellitus or hyperlipoidemia, comprising administering to a subject in need thereof a therapeutically effective amount of the isolated *Saposhnikovia divaricata* polysaccharide obtained by the present invention.

In one preferred embodiment, the method for treating diabetes mellitus or hyperlipoidemia comprises administering to a subject in need thereof a therapeutically effective amount of a pharmaceutical composition comprising a therapeutically effective amount of the *Saposhnikovia divaricata* polysaccharide obtained by the present invention .

In one aspect, the present invention also provides an isolated *Saposhnikovia divaricata* polysaccharide for use in the treatment of diabetes mellitus or hyperlipoidemia.

The treatment of diabetes mellitus as described in the present application comprises lowering the blood glucose level (e.g., lowering the fast blood glucose level), improving glucose tolerance, alleviating pancreatic islet cell damage, increasing insulin release, etc. The treatment of hyperlipoidemia as described in the present application comprises regulating the blood lipid metabolism, and regulating the blood lipid levels (e.g., lowering the level of lipids in the blood, such as lowering the levels of total cholesterol (TC), triglyceride (TG) and low-density lipoprotein cholesterol (LDL-C) in the blood). Additionally, the *Saposhnikovia divaricata* polysaccharide of the present application can also increase the activities of superoxide dismutase (SOD) and glutathione peroxidase (GSH-px) in a subject (e.g., in serum and liver), and lower the content of malondialdehyde (MDA).

The term "treatment" as used herein generally refers to achieving a desired pharmacological and/or physiological effect. The effect can be prophylactic in terms of complete or partial prevention of a disease or symptoms thereof; and/or can be therapeutic in terms of partial or complete stabilization or curing of a disease and/or side effects due to the disease. The term "treatment" as used herein covers any treatment for a patient's disease, including: (a) preventing a disease or symptom in a patient who is susceptible to the disease or symptom but has not yet been diagnosed with the disease; (b) suppressing the symptom of the disease, i.e., preventing its progression; or (c) alleviating the symptom of the disease, i.e., resulting in regression of the disease or symptom.

Unless otherwise specified, the percentages, proportions, ratios or parts used in the present application are by volume. The volume : weight ratio used in the present application is a volume-to-weight ratio as calculated in milliliter/gram (or liter/kilogram). The concentration used in the present application is a volume concentration.

**BRIEF DESCRIPTION OF THE DRAWINGS**

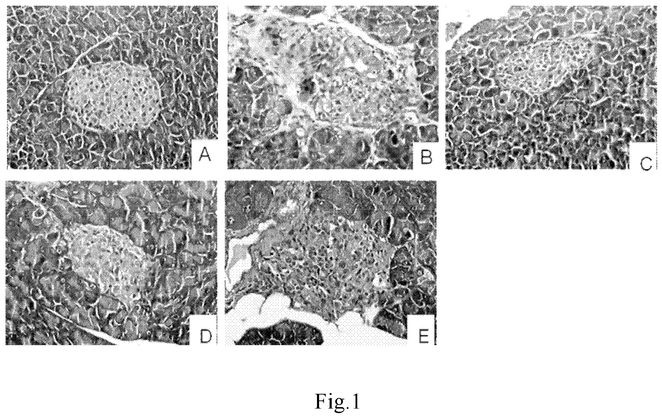

Fig. 1: Effect of *Saposhnikovia divaricata* polysaccharide on pancreatic islet tissue in mice with STZ-induced diabetes mellitus. Fig. 1A: Normal control group; Fig. 1B: STZ 120 mg/kg group; Fig. 1C: Glibenclamide 25 mg/kg group; Fig. 1D: *Saposhnikovia divaricata* polysaccharide 50 mg/kg group; Fig. 1E: *Saposhnikovia divaricata* polysaccharide 200 mg/kg group. STZ: streptozotocin.

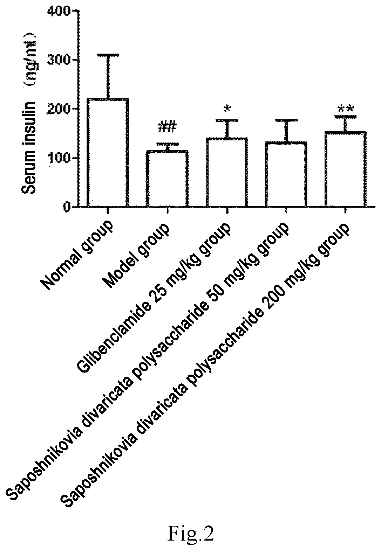

Fig. 2: Effect of *Saposhnikovia divaricata* polysaccharide on serum insulin level in mice with STZ-induced diabetes mellitus ($\bar{x}\pm$S.D, n=10). ##$P<0.01$ (tested by the LSD method), as compared with the normal control group (Normal group); *$P<0.05$, **$P<0.01$ (tested by the LSD method), as compared with the model group (STZ 120 mg/kg); GLI: glibenclamide; SDP: *Saposhnikovia divaricata* polysaccharide; STZ: streptozotocin.

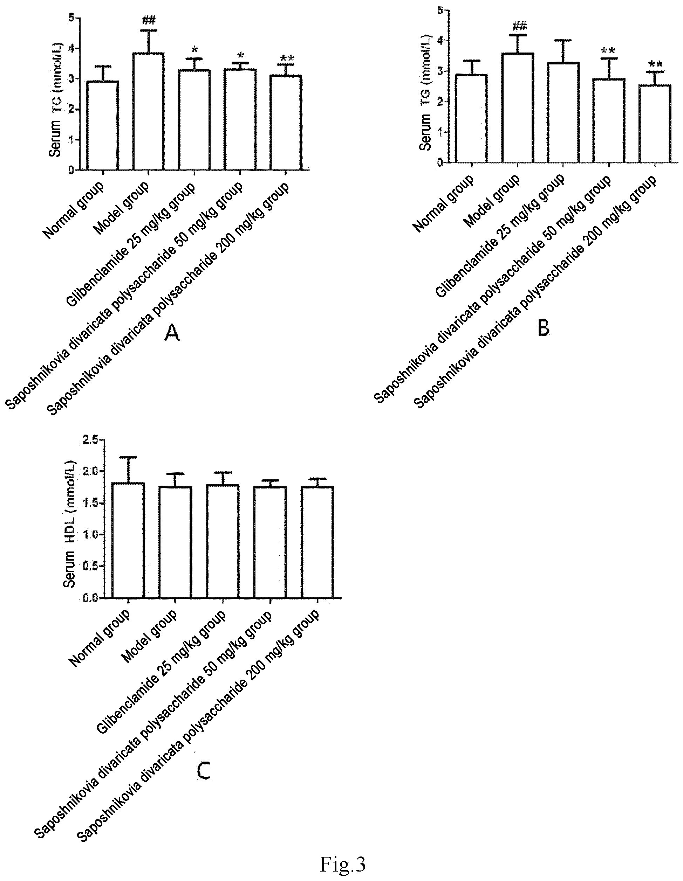

Fig. 3: Effect of *Saposhnikovia divaricata* polysaccharide on serum lipids in mice with STZ-induced diabetes mellitus ($\overline{x}\pm$S.D, n=10). ##P<0.01 (tested by the LSD method), as compared with the normal control group (Normal group); *P<0.05, **P<0.01 (tested by the LSD method), as compared with the model group (STZ 120 mg/kg); GLI: glibenclamide; SDP: *Saposhnikovia divaricata* polysaccharide; STZ: streptozotocin.

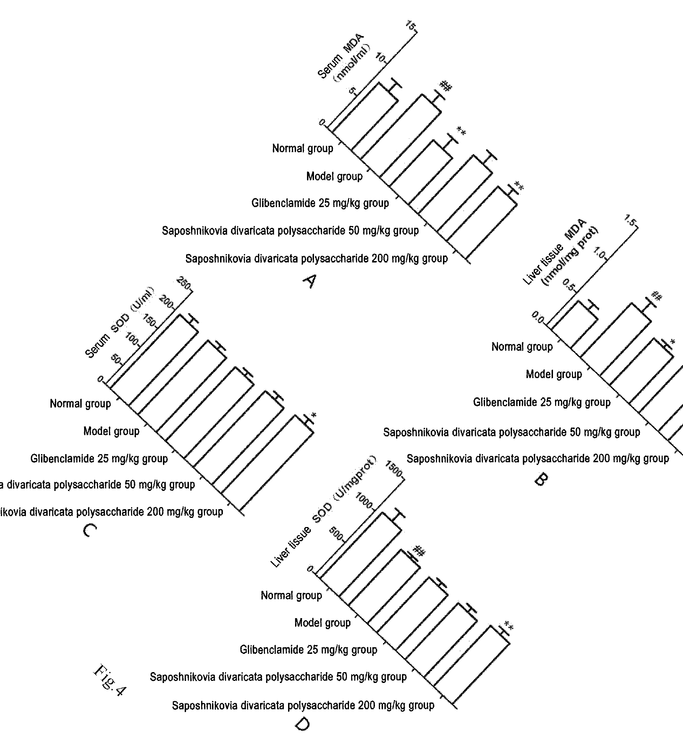

Fig. 4: Effect of *Saposhnikovia divaricata* polysaccharide on MDA content and SOD activity in mice with STZ-induced diabetes mellitus ($\overline{x}\pm$S.D, n=10). ##P<0.01 (tested by the LSD method), as compared with the normal control group (Normal group); *P<0.05, **P<0.01 (tested by the LSD method), as compared with the model group (STZ 120 mg/kg); GLI: glibenclamide; SDP: *Saposhnikovia divaricata* polysaccharide; STZ: streptozotocin.

Fig.: 5: Effect of *Saposhnikovia divaricata* polysaccharide on blood lipid level in mice with hyperlipoidemia ($\overline{x}\pm$S.D, n=10). #P<0.05, ##P<0.01 (tested by the LSD method), as compared with the normal control group (Normal group); *P<0.05, **P<0.01 (tested by the LSD method), as compared with the high-fat model group.

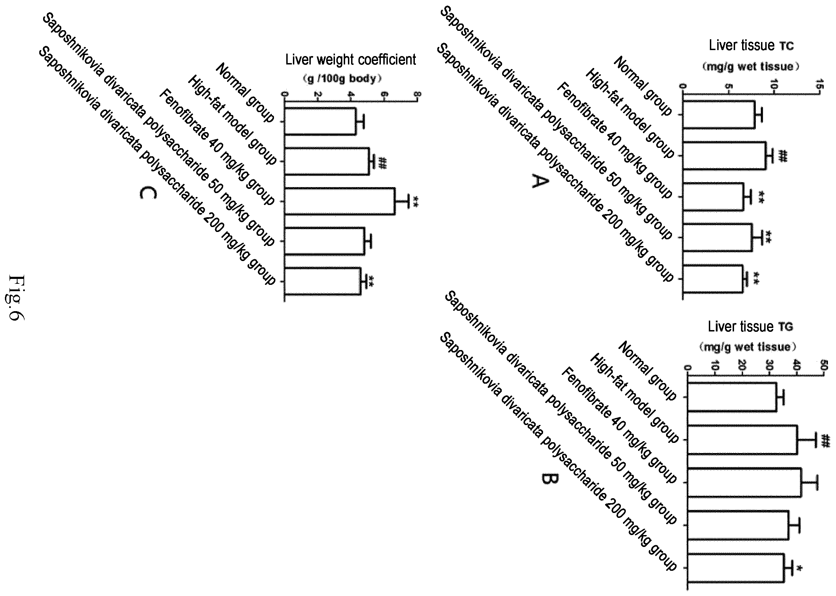

Fig. 6: Effects of *Saposhnikovia divaricata* polysaccharide on liver TC and TG contents and liver weight coefficient in mice with hyperlipoidemia ($\overline{x}\pm$S.D, n=10). ##P<0.01 (tested by the LSD method), as compared with the normal control group (Normal group); *P<0.05, **P<0.01 (tested by the LSD method), as compared with the high-fat model group.

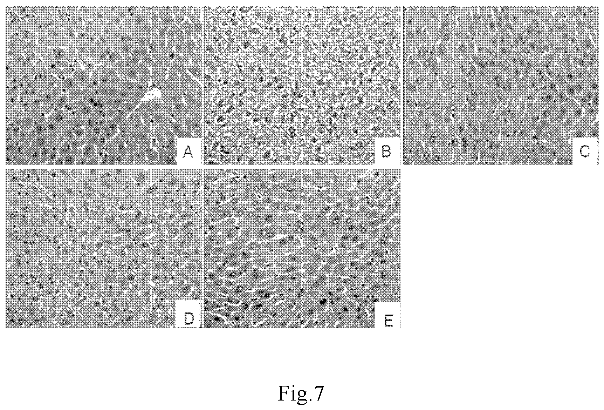

Fig. 7: Effect of *Saposhnikovia divaricata* polysaccharide on liver morphology of mice with hyperlipoidemia. Fig. 7A: Normal control group; Fig. 7B: High-fat model group; Fig. 7C: Lipanthyl 40 mg/kg group; Fig. 7D: *Saposhnikovia divaricata* polysaccharide 50 mg/kg group; Fig. 7E: *Saposhnikovia divaricata* polysaccharide 200 mg/kg group.

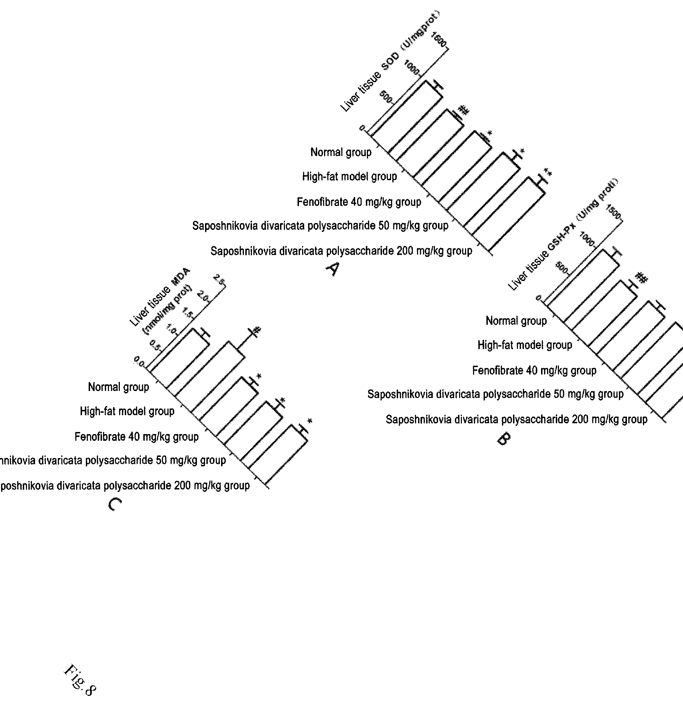

Fig. 8: Effects of *Saposhnikovia divaricata* polysaccharide on liver SOD and GSH-px activities and MDA content in mice with hyperlipoidemia ($\overline{x}\pm$S.D, n=10). #P<0.05, ##P<0.01 (tested by the LSD method), as compared with the normal control group (Normal group); *P<0.05, **P<0.01 (tested by the LSD method), as compared with the high-fat model group.

## DETAILED DESCRIPTION OF THE INVENTION

The present application will demonstrate the beneficial effects of the present application through Examples below. Those skilled in the art will know that these Examples are illustrative and not limiting. These Examples will not limit the scope of the present application in any way. The experimental operations described in the following Examples are conventional operations, unless otherwise specified; and the reagents and materials are commercially available, unless otherwise specified.

Main Reagents and Materials

*Saposhnikovia divaricata* decoction pieces were purchased from the traditional Chinese medicinal material market in Bozhou, Anhui, and produced in Anguo. 95% ethanol, hydrochloric acid, sodium hydroxide, Coomassie brilliant blue, sulfuric acid, phenol, barium chloride, trifluoroacetic acid, sodium borohydride, dimethylsulfoxide, etc. were purchased from Sinopharm Chemical Reagent Co., Ltd. The controls of *L*-arabinose (*L*-Ara), *D*-mannose (*D*-Man), *D*-glucose (*D*-Glc), *D*-galactose (*D*-Gal) and *D*-galacturonic acid (*D*-GalA), and 1-phenyl-3-methyl-5-pyrazolone (PMP) were purchased from Sigma.

Main Instruments

Model 1260 High Performance Liquid Chromatograph (DAD and RID detectors, Agilent, US); DAWN HELEOS-II 18-Angle Laser Light Scattering Spectrometer (Wayyat, US); Model 7890B Gas Chromatograph-Mass Spectrometer (Agilent, US); Infinite M200 Microplate Reader (Tecan, US).

Example 1: Preparation of *Saposhnikovia divaricata* Polysaccharide

(1) 6 L of distilled water was added to 300 g of *Saposhnikovia divaricata* decoction pieces. The *Saposhnikovia divaricata* decoction pieces were extracted with distilled water at 90 °C for 2 h to give an extract. After separation of the extract, the extraction was repeated twice, with 6 L of distilled water for 2 h each time. The resulting extracts were combined and concentrated to 2 L, to give a concentrated extract.

(2) Ethanol was added to the concentrated extract to give a mixture with an ethanol concentration of 20%, and the mixture was centrifuged to give a supernatant.

(3) Ethanol was added to the supernatant to give a mixture with an ethanol concentration of 80%, and the mixture was centrifuged to give a precipitate.

(3') 0.5 L of distilled water was added to the precipitate to dissolve it. Then, ethanol was added again to give a mixture with an ethanol concentration of 80%, and the mixture was centrifuged to give a precipitate.

(4) The resulting precipitate was dried to obtain 6 g of *Saposhnikovia divaricata* polysaccharide, with a yield of 2%.

Example 2: Preparation of *Saposhnikovia divaricata* Polysaccharide

(1) 9 L of distilled water was added to 300 g of *Saposhnikovia divaricata* decoction pieces. The *Saposhnikovia divaricata* decoction pieces were extracted with distilled water at 40 °C for 4 h to give an extract. After separation of the extract, the extraction was repeated three times, with 9 L of distilled water for 4 h each time. The resulting extracts were combined and concentrated to 2 L, to give a concentrated extract.

(2) Methanol was added to the concentrated extract to give a mixture with a methanol concentration of 30%, and the mixture was centrifuged to give a supernatant.

(3) Methanol was added to the supernatant to give a mixture with a methanol concentration of 90%, and the mixture was centrifuged to give a precipitate.

(3') 0.5 L of distilled water was added to the precipitate to dissolve it. Then, methanol was added again to give a mixture with a methanol concentration of 90%, and the mixture was centrifuged to give a precipitate. The operation of step (3') was repeated twice.

(4) The resulting precipitate was dried to obtain 4.8 g of *Saposhnikovia divaricata* polysaccharide, with a yield of 1.6%.

Example 3: Preparation of *Saposhnikovia divaricata* Polysaccharide

(1) 3 L of distilled water was added to 300 g of *Saposhnikovia divaricata* decoction pieces. The *Saposhnikovia divaricata* decoction pieces were extracted with distilled water at 90 °C for 2 h to give an extract. After separation of the extract, the extraction was repeated 3 times, with 3 L of distilled water for 2 h each time. The resulting extracts were combined and concentrated to 2 L, to give a concentrated extract.

(2) Ethanol was added to the concentrated extract to give a mixture with an ethanol concentration of 15%, and the mixture was centrifuged to give a supernatant.

(3) Ethanol was added to the supernatant to give a mixture with an ethanol concentration of 80%, and the mixture was centrifuged to give a precipitate.

(3') 0.5 L of distilled water was added to the precipitate to dissolve it. Then, ethanol was added again to give a mixture with an ethanol concentration of 80%, and the mixture was centrifuged to give a precipitate. The operation of step (3') was repeated once.

(4) The resulting precipitate was dried to obtain 6.2 g of *Saposhnikovia divaricata* polysaccharide, with a yield of 2.1%.

Example 4: Preparation of *Saposhnikovia divaricata* Polysaccharide

(1) 2.4 L of distilled water was added to 300 g of *Saposhnikovia divaricata* decoction pieces. The *Saposhnikovia divaricata* decoction pieces were extracted with distilled water at 100 °C for 1 h to give an extract. After separation of the extract, the extraction was repeated 4 times, with 2.4 L of distilled water for 1 h each time. The resulting extracts were combined and concentrated to 2 L, to give a concentrated extract.

(2) Propanol was added to the concentrated extract to give a mixture with a propanol concentration of 20%, and the mixture was centrifuged to give a supernatant.

(3) Propanol was added to the supernatant to give a mixture with a propanol concentration of 70%, and the mixture was centrifuged to give a precipitate.

(3') 0.5 L of distilled water was added to the precipitate to dissolve it. Then, propanol was added again to give a mixture with a propanol concentration of 70%, and the mixture was centrifuged to give a precipitate.

(4) The resulting precipitate was dried to obtain 4.9 g of *Saposhnikovia divaricata* polysaccharide, with a yield of 1.6%.

Example 5: Preparation of *Saposhnikovia divaricata* Polysaccharide

(1) 6 L of distilled water was added to 300 g of *Saposhnikovia divaricata* decoction pieces. The *Saposhnikovia divaricata* decoction pieces were extracted with distilled water at 60 °C for 3 h to give an extract. After separation of the extract, the extraction was repeated twice, with 6 L of distilled water for 3 h each time. The resulting extracts were combined and concentrated to 2 L, to give a concentrated extract.

(2) Propanol was added to the concentrated extract to give a mixture with a propanol concentration of 20%, and the mixture was centrifuged to give a supernatant.

(3) Propanol was added to the supernatant to give a mixture with a propanol concentration of 75%, and the mixture was centrifuged to give a precipitate.

(3') 0.5 L of distilled water was added to the precipitate to dissolve it. Then, propanol was added again to give a mixture with a propanol concentration of 75%, and the mixture was centrifuged to give a precipitate.

(4) The resulting precipitate was dried to obtain 5.2 g of *Saposhnikovia divaricata* polysaccharide, with a yield of 1.7%.

Example 6: Preparation of *Saposhnikovia divaricata* Polysaccharide

(1) 6 L of distilled water was added to 300 g of *Saposhnikovia divaricata* decoction pieces. The *Saposhnikovia divaricata* decoction pieces were extracted with distilled water at 80 °C for 2 h to give an extract. After separation of the extract, the extraction was repeated twice, with 6 L of distilled water for 2 h each time. The resulting extracts were combined and concentrated to 2 L, to give a concentrated extract.

(2) Ethanol was added to the concentrated extract to give a mixture with an ethanol concentration of 25%, and the mixture was centrifuged to give a supernatant.

(3) Ethanol was added to the supernatant to give a mixture with an ethanol concentration of 80%, and the mixture was centrifuged to give a precipitate.

(4) The resulting precipitate was dried to obtain 5.8 g of *Saposhnikovia divaricata* polysaccharide, with a yield of 1.9%.

Example 7: Preparation of *Saposhnikovia divaricata* Polysaccharide

(1) 9 L of distilled water was added to 300 g of *Saposhnikovia divaricata* decoction pieces. The *Saposhnikovia divaricata* decoction pieces were extracted with distilled water at 70 °C for 2 h to give an extract. After separation of the extract, the extraction was repeated three times, with 9 L of distilled water for 2 h each time. The resulting extracts were combined and concentrated to 2 L, to give a concentrated extract.

(2) Ethanol was added to the concentrated extract to give a mixture with an ethanol concentration of 20%, and the mixture was centrifuged to give a supernatant.

(3) Ethanol was added to the supernatant to give a mixture with an ethanol concentration of 85%, and the mixture was centrifuged to give a precipitate.

(3') 0.5 L of distilled water was added to the precipitate to dissolve it. Then, ethanol was added again to give a mixture with an ethanol concentration of 85%, and the mixture was centrifuged to give a precipitate.

(4) The resulting precipitate was dried to obtain 5.7 g of *Saposhnikovia divaricata* polysaccharide, with a yield of 1.9%.

Example 8: Preparation of *Saposhnikovia divaricata* Polysaccharide

(1) 6 L of distilled water was added to 300 g of *Saposhnikovia divaricata* decoction pieces. The *Saposhnikovia divaricata* decoction pieces were extracted with distilled water at 95 °C for 1 h to give an extract. After separation of the extract, the extraction was repeated once. The resulting extracts were combined and concentrated to 2 L, to give a concentrated extract.

(2) Ethanol was added to the concentrated extract to give a mixture with an ethanol concentration of 20%, and the mixture was centrifuged to give a supernatant.

(3) Ethanol was added to the supernatant to give a mixture with an ethanol concentration of 80%, and the mixture was centrifuged to give a precipitate.

(3') 0.5 L of distilled water was added to the precipitate to dissolve it. Then, ethanol was added again to give a mixture with an ethanol concentration of 80%, and the mixture was centrifuged to give a precipitate.

(4) The resulting precipitate was dried to obtain 6.1 g of *Saposhnikovia divaricata* polysaccharide, with a yield of 2.0%.

Example 9: Structural identification of *Saposhnikovia divaricata* Polysaccharide

(1) Determination of the contents of total sugars, uronic acids, proteins and sulfate groups

The contents of total sugars of the *Saposhnikovia divaricata* polysaccharides obtained from Examples 1-8 were determined by the sulfuric acid-phenol method (see Zeqing Zhang, Purification, structural analysis and biological activity study of Saposhnikovia divaricata polysaccharides, Master's Thesis of Shaanxi Normal University, 2008, p. 77).

(2) The content of uronic acids of the *Saposhnikovia divaricata* polysaccharides obtained from Examples 1-8 were determined by the m-hydroxybiphenyl method (see Lin Gao, Quantitative Determination of Uronic Acid in MCP, Chemical Industry and Engineering, 2005, 22(6): 487-489).

(3) The content of proteins of the *Saposhnikovia divaricata* polysaccharides obtained from Examples 1 to 8 were determined by the Coomassie brilliant blue method (see Jie Zhang, Determination of the basic content of Phellodendron amurense polysaccharides before and after stir-heating with a salt solution and its effect on immune function, Liaoning Journal of Traditional Chinese Medicine, 2017, 44(6): 1263-1267).

(4) The content of sulfate groups of the *Saposhnikovia divaricata* polysaccharides obtained from Examples 1 to 8 were determined by the barium sulfate-turbidimetry (see Qian Chen, Determination of the sulfate content in fucoidan by barium sulfate-turbidimetry, Journal of Pharmaceutical Practice, 2012, 30(2): 118-120).

The measurement results are shown in Table 1 below.

Table 1

| Example No. | Content of total sugars (%) | Content of uronic acids | Content of proteins (%) | Content of sulfate groups |
|---|---|---|---|---|
| 1 | 76.33 | 16.41 | 1.17 | n.d. |
| 2 | 71.48 | 18.22 | 2.46 | n.d. |
| 3 | 77.89 | 14.14 | 1.33 | n.d. |
| 4 | 72.35 | 16.72 | 2.07 | n.d. |
| 5 | 75.53 | 13.66 | 1.24 | n.d. |
| 6 | 76.47 | 17.36 | 2.21 | n.d. |
| 7 | 74.92 | 18.41 | 1.53 | n.d. |
| 8 | 79.62 | 13.74 | 1.83 | n.d. |

(5) Determination of weight average molecular weights

The weight average molecular weights of the *Saposhnikovia divaricata* polysaccharides obtained from Examples 1-8 were determined by the multi-angle laser light scattering method (see Houqiang Ding, Determination of molecular weight and distribution of hyaluronic acid by combination of multi-angle laser light scattering spectrometer and size exclusion chromatography, Food and Drug, 2009, 11(3): 24-26).

Assay Method

10 mg of the sample to be tested was placed in a 1.5 mL centrifuge tube. Then, 1 mL of deionized water was added to dissolve the sample. The centrifuge tube was centrifuged at 14000 rpm for 10 min to obtain a supernatant. The supernatant was assayed on an Agilent 1260 HPLC chromatograph to determine the weight average molecular weight.

Chromatographic Conditions:

Chromatographic column: XBridge Protein BEH SEC 200 Å Column (3.5 $\mu$m, 7.8×300 mm); column temperature: 25 °C; RID temperature: 35 °C; mobile phase: 0.1 mol/L NaOAc solution; flow rate: 0.5 mL/min; injection volume: 30 $\mu$L.
The measurement results are shown in Table 2:

Table 2

| Example No. | Weight average molecular weight/Da |
|---|---|
| 1 | $3.5 \times 10^5$ |
| 2 | $1.8 \times 10^5$ |
| 3 | $4.4 \times 10^5$ |
| 4 | $2.7 \times 10^5$ |
| 5 | $8.5 \times 10^4$ |
| 6 | $2.4 \times 10^5$ |
| 7 | $1.3 \times 10^5$ |
| 8 | $3.4 \times 10^5$ |

(6) Analysis of the monosaccharide composition

2 mg of each of the *Saposhnikovia divaricata* polysaccharides obtained from Examples 1-8 was dissolved in 1 mL of a 3 mol/L solution of trifluoroacetic acid (TFA) in water in an ampoule, and then the ampoule was sealed. The *Saposhnikovia divaricata* polysaccharide in the ampoule was hydrolyzed at 105 °C for 4 h. After the water in the ampoule was evaporated under reduced pressure to dryness, 2 mL of methanol was added to the ampoule and then evaporated to dryness. The addition of ethanol and evaporation to dryness were repeated twice to remove TFA. Then, 100 μL of water was added to the ampoule to afford a sample of the polysaccharide that was completely hydrolyzed under an acidic condition.

Then, a suitable amount of a monosaccharide control was weighed to prepare a mother solution at a concentration of 1 mg/mL. 10 μL of the mother solution was pipetted and diluted to the constant volume of 100 μL.

Derivatization: To 50 μL of the control solution were added 100 μL of a 0.3 mol/L NaOH solution, 120 μL of a 0.5 mol/L solution of 1-phenyl-3-methyl-5-pyrazolone in methanol sequentially, and mixed well to obtain a mixed solution. The mixed solution was reacted at 70 °C for 60 min. After completion of the reaction, the solution was cooled to room temperature, a suitable amount of 0.3 mol/L HCl was added to adjust the pH to neutral, the solution was extracted with 1 mL of chloroform, and then the organic phase was discarded. 50 μL of the sample of the polysaccharide that was completely hydrolyzed under an acidic condition was subjected to the same derivatization according to the above method.

Chromatographic Conditions:

Agilent Eclipse XDB-C18 chromatographic column; mobile phase: 0.1 mol/L phosphate buffer (pH=6.7) : acetonitrile (v/v=83:17); column temperature: 25 °C; detection wavelength: 245 nm; flow rate: 1.0 mL/min; injection volume: 10 μL.

The measurement results are shown in Table 3 below:

Table 3

| Example No. | Molar ratio (*L*-Ara: *D*-GalA: *D*-Man: *D*-Glc: *D*-Gal) |
|---|---|
| 1 | 5:6:2:23:5 |
| 2 | 6:6:7:24:2 |
| 3 | 6:2:5:20:3 |
| 4 | 4:6:3:25:4 |
| 5 | 5:8:3:25:4 |
| 6 | 3:6:5:23:2 |
| 7 | 4:7:4:21:3 |
| 8 | 3:7:5:22:4 |

(7) Methylation Analysis

The *Saposhnikovia divaricata* polysaccharides of Examples 1-8 were respectively methylated by the method as described in the reference (Jinian Fang, Methylation analysis methods of polysaccharides, Foreign Medical Sciences (Section of Pharmacology), 1986, (4): 222-226). The methylated product was depolymerized with 90% formic acid, and fully hydrolyzed with 2 mol/L TFA to afford methylated monosaccharides. Then, the resulting methylated monosaccharides were reduced with $NaBH_4$ and acetylated with acetic anhydride to generate alditol acetate derivatives of the methylated monosaccharides. Then, the derivatives were subjected to GC-MS analysis.

It could be determined from the results of the methylation analysis that the *Saposhnikovia divaricata* polysaccharides of Examples 1-8 comprised the following monosaccharides: 1,4-*L*-arabinose, 1,3,4-*L*-arabinose, terminal *D*-galacturonic acid, 1,6-*D*-mannose, 1,4-*D*-glucose, 1,3,6-*D*-glucose, terminal *D*-galactose, and 1,4-*D*-galactose. The methylation analysis results are shown in Tables 4-11.

**Table 4 Methylation analysis results of the *Saposhnikovia divaricata* polysaccharide obtained from Example 1**

| Methylated sugar residue | Monosaccharide | Molar ratio |
|---|---|---|
| 2,3-Me$_2$-L-Ara | 1,4-L-arabinose | 1 |
| 2-Me-L-Ara | 1,3,4-L-arabinose | 4 |
| 2,3,4-Me$_3$-D-GalA | terminal D-galacturonic acid | 6 |

(continued)

| Methylated sugar residue | Monosaccharide | Molar ratio |
|---|---|---|
| $2,3,4\text{-Me}_3\text{-D-Man}$ | 1,6-D-mannose | 2 |
| $2,3,6\text{-Me}_3\text{-D-Glc}$ | 1,4-D-glucose | 20 |
| $2,4\text{-Me}_2\text{-D-Glc}$ | 1,3,6-D-glucose | 3 |
| $2,3,4,6\text{-Me}_4\text{-D-Gal}$ | terminal -D-galactose | 1 |
| $2,3,6\text{-Me}_3\text{-D-Gal}$ | 1,4-D-galactose | 4 |

**Table 5 Methylation analysis results of the *Saposhnikovia divaricata* polysaccharide obtained from Example 2**

| Methylated sugar residue | Monosaccharide | Molar ratio |
|---|---|---|
| $2,3\text{-Me}_2\text{-L-Ara}$ | 1,4-L-arabinose | 1 |
| $2\text{-Me-L-Ara}$ | 1,3,4-L-arabinose | 1 |
| $2,3,4\text{-Me}_3\text{-D-GalA}$ | terminal D-galacturonic acid | 6 |
| $2,3,4\text{-Me}_3\text{-D-Man}$ | 1,6-D-mannose | 9 |
| $2,3,6\text{-Me}_3\text{-D-Glc}$ | 1,4-D-glucose | 10 |
| $2,4\text{-Me}_2\text{-D-Glc}$ | 1,3,6-D-glucose | 2 |
| $2,3,4,6\text{-Me}_4\text{-D-Gal}$ | terminal -D-galactose | 1 |
| $2,3,6\text{-Me}_3\text{-D-Gal}$ | 1,4-D-galactose | 1 |

**Table 6 Methylation analysis results of the *Saposhnikovia divaricata* polysaccharide obtained from Example 3**

| Methylated sugar residue | Monosaccharide | Molar ratio |
|---|---|---|
| $2,3\text{-Me}_2\text{-L-Ara}$ | 1,4-L-arabinose | 1 |
| $2\text{-Me-L-Ara}$ | 1,3,4-L-arabinose | 3 |
| $2,3,4\text{-Me}_3\text{-D-GalA}$ | terminal D-galacturonic acid | 7 |
| $2,3,4\text{-Me}_3\text{-D-Man}$ | 1,6-D-mannose | 4 |
| $2,3,6\text{-Me}_3\text{-D-Glc}$ | 1,4-D-glucose | 24 |
| $2,4\text{-Me}_2\text{-D-Glc}$ | 1,3,6-D-glucose | 4 |
| $2,3,4,6\text{-Me}_4\text{-D-Gal}$ | terminal -D-galactose | 1 |
| $2,3,6\text{-Me}_3\text{-D-Gal}$ | 1,4-D-galactose | 4 |

**Table 7 Methylation analysis results of the *Saposhnikovia divaricata* polysaccharide obtained from Example 4**

| Methylated sugar residue | Monosaccharide | Molar ratio |
|---|---|---|
| $2,3\text{-Me}_2\text{-L-Ara}$ | 1,4-L-arabinose | 3 |
| $2\text{-Me-L-Ara}$ | 1,3,4-L-arabinose | 10 |
| $2,3,4\text{-Me}_3\text{-D-GalA}$ | terminal D-galacturonic acid | 9 |
| $2,3,4\text{-Me}_3\text{-D-Man}$ | 1,6-D-mannose | 3 |
| $2,3,6\text{-Me}_3\text{-D-Glc}$ | 1,4-D-glucose | 16 |

(continued)

| Methylated sugar residue | Monosaccharide | Molar ratio |
|---|---|---|
| 2,4-Me$_2$-D-Glc | 1,3,6-D-glucose | 9 |
| 2,3,4,6-Me$_4$-D-Gal | terminal-D-galactose | 5 |
| 2,3,6-Me$_3$-D-Gal | 1,4-D-galactose | 9 |

**Table 8 Methylation analysis results of the *Saposhnikovia divaricata* polysaccharide obtained from Example 5**

| Methylated sugar residue | Monosaccharide | Molar ratio |
|---|---|---|
| 2,3-Me$_2$-L-Ara | 1,4-L-arabinose | 4 |
| 2-Me-L-Ara | 1,3,4-L-arabinose | 2 |
| 2,3,4-Me$_3$-D-GalA | terminal D-galacturonic acid | 6 |
| 2,3,4-Me$_3$-D-Man | 1,6-D-mannose | 7 |
| 2,3,6-Me$_3$-D-Glc | 1,4-D-glucose | 22 |
| 2,4-Me$_2$-D-Glc | 1,3,6-D-glucose | 2 |
| 2,3,4,6-Me$_4$-D-Gal | terminal -D-galactose | 1 |
| 2,3,6-Me$_3$-D-Gal | 1,4-D-galactose | 1 |

**Table 9 Methylation analysis results of the *Saposhnikovia divaricata* polysaccharide obtained from Example 6**

| Methylated sugar residue | Monosaccharide | Molar ratio |
|---|---|---|
| 2,3-Me$_2$-L-Ara | 1,4-L-arabinose | 1 |
| 2-Me-L-Ara | 1,3,4-L-arabinose | 2 |
| 2,3,4-Me$_3$-D-GalA | terminal D-galacturonic acid | 6 |
| 2,3,4-Me$_3$-D-Man | 1,6-D-mannose | 5 |
| 2,3,6-Me$_3$-D-Glc | 1,4-D-glucose | 18 |
| 2,4-Me$_2$-D-Glc | 1,3,6-D-glucose | 5 |
| 2,3,4,6-Me$_4$-D-Gal | terminal -D-galactose | 1 |
| 2,3,6-Me$_3$-D-Gal | 1,4-D-galactose | 1 |

**Table 10 Methylation analysis results of the *Saposhnikovia divaricata* polysaccharide obtained from Example 7**

| Methylated sugar residue | Monosaccharide | Molar ratio |
|---|---|---|
| 2,3-Me$_2$-L-Ara | 1,4-L-arabinose | 1 |
| 2-Me-L-Ara | 1,3,4-L-arabinose | 3 |
| 2,3,4-Me$_3$-D-GalA | terminal D-galacturonic acid | 7 |
| 2,3,4-Me$_3$-D-Man | 1,6-D-mannose | 4 |
| 2,3,6-Me$_3$-D-Glc | 1,4-D-glucose | 20 |
| 2,4-Me$_2$-D-Glc | 1,3,6-D-glucose | 1 |
| 2,3,4,6-Me$_4$-D-Gal | terminal -D-galactose | 1 |

(continued)

| Methylated sugar residue | Monosaccharide | Molar ratio |
|---|---|---|
| 2,3,6-Me$_3$-D-Gal | 1,4-D-galactose | 2 |

**Table 11 Methylation analysis results of the *Saposhnikovia divaricata* polysaccharide obtained from Example 8**

| Methylated sugar residue | Monosaccharide | Molar ratio |
|---|---|---|
| 2,3-Me$_2$-L-Ara | 1,4-L-arabinose | 3 |
| 2-Me-L-Ara | 1,3,4-L-arabinose | 2 |
| 2,3,4-Me$_3$-D-GalA | terminal D-galacturonic acid | 6 |
| 2,3,4-Me$_3$-D-Man | 1,6-D-mannose | 3 |
| 2,3,6-Me$_3$-D-Glc | 1,4-D-glucose | 20 |
| 2,4-Me$_2$-D-Glc | 1,3,6-D-glucose | 5 |
| 2,3,4,6-Me$_4$-D-Gal | terminal -D-galactose | 2 |
| 2,3,6-Me$_3$-D-Gal | 1,4-D-galactose | 2 |

Example 10: Experiment on the effect in lowering blood glucose

Experimental drug: the *Saposhnikovia divaricata* polysaccharide from Example 1 was administered at doses of 50 mg/kg (low dose), and 200 mg/kg (high dose), respectively.

Experimental reagents: glucose assay kit, provided by Shanghai Rongsheng Biopharmaceutical Co., Ltd.; insulin ELISA assay kit, provided by Shanghai Westang Industrial Co., Ltd.; TC, TG, HDL-C, SOD, MDA and Coomassie brilliant blue protein assay kits were all provided by Nanjing Jiancheng Bioengineering Institute.

Experimental animals: healthy male Kunming mice of clean grade (weighed 18-22 g, provided by Shanghai SLAC Laboratory Animal Co., Ltd.).

Experimental instruments: high-speed freezing centrifuge, produced by Eppendorf, Germany; electronic balance, produced by Mettler-Toledo; multifunctional microplate reader, produced by Berten Instrument Co., Ltd., US.

Experimental method: The mice were kept at a temperature of 20±2°C and a humidity of 50±5% under 12 hours of light and 12 hours of darkness for 3 days, during which the mice were allowed to access to food and water *ad arbitrium.* 10 healthy mice were selected as the normal control group (Control). The remaining mice were intraperitoneally injected with a streptozotocin (STZ) solution at 0.2 ml/10 g body weight. 72 h later, the fast blood glucose (FBG) of the mice was measured. The mice having an FBG value higher than 11.1 mmol/L and lower than 25 mmol/L were selected as diabetes mellitus model mice. The diabetes mellitus model mice were randomly grouped into a model group (STZ 120 mg/kg), a low-dose *Saposhnikovia divaricata* polysaccharide group (SDP 50 mg/kg), a high-dose *Saposhnikovia divaricata* polysaccharide (SDP 200 mg/kg) group, and a positive drug (Glibenclamide, GLI 25 mg/kg) control group, 10 mice per group. For each dosing group, the mice were dosed by oral gavage at 0.2 ml/10 g body weight at 8:00 am every day. The normal control group and the model group were given an equal volume of distilled water. During the experiment, the mice in each group were allowed to access to water *ad arbitrium.* The FBG and body weight were measured every 10 days. 30 days after the dosing, the mice were treated, and the following indexes were measured.

(1) Glucose tolerance: The mice were fasted at 8:00 the day before the end of the experiment. After 4 h, blood was collected from the orbit, and FBG was measured as the blood glucose level before administration of glucose (0 h). Then, all mice were intraperitoneally injected with a 10% glucose solution at 0.2 ml/10 g body weight. 0.5 hour (0.5 h) and 2 hours (2 h) after intraperitoneal injection of glucose, blood was collected from the orbit, and the blood glucose value was measured. The area under the blood glucose curve (AUC) was calculated according to the following equation:

Area under the blood glucose curve＝

$$\frac{(\text{glucose at 0h} + \text{glucose at 0.5h}) \times 0.5}{2} + \frac{(\text{glucose at 2h} + \text{glucose at 0.5h}) \times 1.5}{2}$$

(2) The mice were fasted with free access to water at 22:00 the day before the end of the experiment, and the experiment started at 8:00. Blood was collected from the orbit, and serum was obtained from the blood by centrifugation. The serum levels of insulin, total cholesterol (TC), triglyceride (TG) and high-density lipoprotein cholesterol (HDL-C), as well as the SOD activity and MDA content were measured. A part of the liver tissue of the mice was took, homogenized, and measured for the liver SOD (superoxide dismutase) activity and MDA (malondialdehyde) content. A part of the pancreatic tissue of the mice was took, fixed with 10% formaldehyde, and examined for morphology.

Experimental Results:

(1) Effect of *Saposhnikovia divaricata* polysaccharide on fast blood glucose and glucose tolerance in mice with STZ-induced diabetes mellitus.

As shown in Table 12, the blood glucose level of the model group mice was significantly increased (P<0.01) as compared with the normal control group. As compared with the model group, the *Saposhnikovia divaricata* polysaccharide could significantly lower the blood glucose level of diabetic mice (P<0.05).

Table 12 Effect of *Saposhnikovia divaricata* polysaccharide in lowering fast blood glucose (FBG) in mice with diabetes mellitus induced by streptozotocin (STZ) ($\bar{x}\pm$S.D, n=10)

| group | FBG (mmol/l) | | | |
| --- | --- | --- | --- | --- |
| | Day 0 | Day 10 | Day 20 | Day 30 |
| Normal control group | 6.81±1.93 | 6.66±1.11 | 6.78±0.63 | 6.13±2.08 |
| Model group | 12.85±3.20## | 16.63±1.42## | 21.33±1.89## | 22.28±1.52## |
| Positive drug control group GLI (25 mg/kg) | 12.58±2.69 | 15.95±1.57 | 18.56±3.02* | 20.44±2.25* |
| Low dose of *Saposhnikovia divaricata* polysaccharide (SDP 50 mg/kg) | 12.29±3.34 | 14.62±2.86 | 18.36±4.10* | 20.04±2.46* |
| High dose of *Saposhnikovia divaricata* polysaccharide (SDP 200 mg/kg) | 12.12±3.14 | 13.84±3.49* | 18.14±3.36* | 19.56±3.13* |

Note: ##P<0.01 (tested by the LSD method), as compared with the normal control group; *P<0.05 (tested by the LSD method), as compared with the model group. GLI: glibenclamide; SDP: *Saposhnikovia divaricata* polysaccharide; STZ: streptozotocin.

As shown in Table 13, the glucose tolerance of the mice in the model group was significantly lowered, and the area under the blood glucose curve was significantly increased (P<0.01) as compared with the normal control group. As compared with the model group, the *Saposhnikovia divaricata* polysaccharide could significantly improve glucose tolerance (P<0.05 or P<0.01), and significantly reduce the area under the blood glucose curve (P<0.01).

Table 13. Effect of *Saposhnikovia divaricata* polysaccharide in reducing fast blood glucose (FBG) in mice with diabetes mellitus induced by streptozotocin (STZ) ($\bar{x}\pm$S.D, n=10)

| Group | Blood glucose (mmol/l) | | | Area under the curve (AUC) |
| --- | --- | --- | --- | --- |
| | Oh | 0.5h | 2h | |
| Normal control group | 6.13±2.08 | 13.96±1.96 | 8.02±1.69 | 21.51±3.23 |
| Model group | 22.28±1.52## | 30.97±3.07## | 29.61±2.10## | 58.75±4.17## |

(continued)

| Group | Blood glucose (mmol/l) | | | Area under the curve (AUC) |
|---|---|---|---|---|
| | Oh | 0.5h | 2h | |
| Positive drug control group GLI (25 mg/kg) | 20.44±2.25* | 29.63±2.24 | 27.60±2.04* | 40.62±2.86** |
| Low dose of *Saposhnikovia divaricata* polysaccharide (SDP 50 mg/kg) | 20.04±2.46* | 27.47±4.12* | 26.70±2.95* | 39.21±4.18** |
| High dose of *Saposhnikovia divaricata* polysaccharide (SDP 200 mg/kg) | 19.56±3.13* | 27.89±2.97* | 24.11±4.65** | 36.92±5.52** |
| Note: ##P<0.01 (tested by the LSD method), as compared with the normal control group; *P<0.05 (tested by the LSD method), as compared with the STZ (120 mg/kg) group. GLI: glibenclamide; SDP: *Saposhnikovia divaricata* polysaccharide; STZ: streptozotocin. | | | | |

(2) Effect of *Saposhnikovia divaricata* polysaccharide on pancreatic islet tissue and serum insulin level in mice with diabetes mellitus induced by streptozotocin (STZ)

As shown in Fig. 1, the cells in the pancreatic islet tissue of the mice with STZ-induced diabetes mellitus exhibited nuclear pyknosis, hyalinization, inflammatory cell infiltration, and incomplete pancreatic islet morphology, as compared with the normal control group (Fig. 1B). After administration of the *Saposhnikovia divaricata* polysaccharide, as compared with the STZ group, the number of pancreatic islets in the *Saposhnikovia divaricata* polysaccharide group was not significantly lower at low magnification; and at high magnification, the damage of the pancreatic islets of the mice in the *Saposhnikovia divaricata* polysaccharide group was significantly alleviated and the morphology was relatively complete (Figs. ID and IE).

At the same time, the serum insulin level of the mice was measured. The experimental results are shown in Fig. 2. As compared with the normal control group, the serum insulin level of the mice in the STZ group (model group) was significantly decreased (P<0.01). After administration of the *Saposhnikovia divaricata* polysaccharide, as compared with the STZ group, the serum insulin level of the *Saposhnikovia divaricata* polysaccharide (200 mg/kg) high-dose group was significantly increased (P<0.01).

(3) Effect of *Saposhnikovia divaricata* polysaccharide on serum lipids in mice with STZ-induced diabetes mellitus

Lipotoxicity is an important aspect of the pathogenesis of type 2 diabetes mellitus. As shown in Fig. 3, the serum TC and TG levels of the mice in the STZ group (model group) were both significantly increased (P<0.01) as compared with the normal control. After administration of the *Saposhnikovia divaricata* polysaccharide, as compared with the STZ group, the low dose and high dose of the *Saposhnikovia divaricata* polysaccharide could significantly lower serum TC and TG levels in the diabetic mice in a dose-dependent manner (P<0.05 or P<0.01). In addition, the serum HDL-C level was not significantly affected across the groups.

(4) Effects of *Saposhnikovia divaricata* polysaccharide on serum and liver MDA contents and SOD activity in mice with diabetes mellitus induced by streptozotocin (STZ)

In the rat model of STZ-induced diabetes mellitus, a large amount of oxygen free radicals will be produced. Lipids undergo peroxidation under the action of the oxygen free radicals, thereby producing a large amount of aldehydes, alcohols and other substances, of which malondialdehyde (MDA) is a representative substance. The content of malondialdehyde reflects the degree of peroxidation. The activity of SOD reflects the body's ability to scavenge oxygen free radicals. The experimental results are shown in Fig. 4. As compared with the normal control, the contents of MDA in the serum and liver of the mice in the STZ group (model group) were both significantly increased (P<0.01), while the activity of SOD in the liver was significantly reduced (P<0.01). After administration of the *Saposhnikovia divaricata* polysaccharide, the *Saposhnikovia divaricata* polysaccharide could reduce the serum MDA content of diabetic mice as compared with the STZ group. In particular, the high dose of the *Saposhnikovia divaricata* polysaccharide could significantly reduce the serum MDA content of the mice (P<0.01); and the low dose and high dose of the *Saposhnikovia divaricata* polysaccharide could significantly reduce the liver MDA content of the mice (P<0.05). As compared with the STZ group, the *Saposhnikovia divaricata* polysaccharide could significantly increase the serum SOD activity in the mice. In particular, the high dose of the *Saposhnikovia divaricata* polysaccharide could significantly increase the serum SOD activity in the mice (P<0.05); and the low dose and high dose of the *Saposhnikovia divaricata* polysaccharide could increase the liver SOD activity in the mice; particularly, the high dose of the *Saposhnikovia divaricata* polysaccharide could significantly increase the liver SOD activity in the mice (P<0.01).

Experimental conclusion: The *Saposhnikovia divaricata* polysaccharide can significantly lower the fast blood glucose level of mice with STZ-induced diabetes mellitus and significantly increase the glucose tolerance of diabetic mice. Moreover, the *Saposhnikovia divaricata* polysaccharide can also alleviate the damage of pancreatic islets of mice, increase the release of insulin, and significantly reduce the serum TC and TG levels in hyperglycemic mice. The *Saposhnikovia divaricata* polysaccharide can also increase the activity of SOD in serum and liver and reduce the content of MDA.

Example 11: Experiment on the effect of *Saposhnikovia divaricata* polysaccharide in lowering blood lipids

Experimental drug: the *Saposhnikovia divaricata* polysaccharide from Example 1 was administered at doses of 50 mg/kg (low dose) and 200 mg/kg (high dose), respectively.

Experimental reagents: TC, TG, LDL-C, HDL-C, SOD, GSH-px, MDA and Coomassie brilliant blue protein assay kits were all provided by Nanjing Jiancheng Bioengineering Institute.

Experimental animals: healthy male Kunming mice of clean grade (weighed 18-22 g, provided by Shanghai SLAC Laboratory Animal Co., Ltd.).

Experimental instruments: high-speed freezing centrifuge, produced by Eppendorf, Germany; electronic balance, produced by Mettler-Toledo; multifunctional microplate reader, produced by Berten Instrument Co., Ltd., US.

A hyperlipoidemia model was established with reference to the methods as described in the references (Liyan Sun, Zhenliang Liu, Jinxia Sun, et al., Effect of Imperata cylindrica polysaccharide on hypoxia tolerance in mice, China Journal of Hospital Pharmacy, 2008, 28(2): 96-99; Bin Leng, Intervention of immunoregulation and renal fibrosis by Imperata cylindrica polysaccharide in rats with IgA nephropathy, Dissertations of Guilin Medical University, 2013; Shijing Lv, Qicai Long, Deyuan He, et al., Regulation of lymphocyte proliferation and T cell subpopulation by Imperata cylindrica polysaccharide in patients with hepatitis B, [Conference Paper] 2001 - The Second National Academic Conference on Immunology of Traditional Chinese Medicine). Experimental method: The mice were kept at a temperature of $20\pm2°C$ and a humidity of $50\pm5\%$ under 12 hours of light and 12 hours of darkness for 3 days, during which the mice were allowed to access to food and water *ad arbitrium*. The mice were randomly grouped into 5 groups: a normal control group (Normal group), a high-fat model group, a positive drug Lipanthyl (Fenofibrate, 40 mg/kg) group, a low-dose *Saposhnikovia divaricata* polysaccharide group (SDP 50 mg/kg), and a high-dose *Saposhnikovia divaricata* polysaccharide group (SDP 200 mg/kg), 10 animals per group. For each dosing group, different doses of the drugs were administered at 0.2 ml/10 g body weight at 8:00 to 9:00 every day. The normal control group and the high-fat model group were given an equal volume of distilled water. Except for the normal control group, the mice in each group were given a high-fat diet (containing 20% lard oil, 10% cholesterol, 0.2% propylthiouracil, 20% propylene glycol, and 20% Tween-80) by oral gavage at 0.2 ml/10 g body weight at 14:00 to 15:00 every day for 3 consecutive weeks, to investigate the effect of the *Saposhnikovia divaricata* polysaccharide in preventing hyperlipoidemia. At the end of the experiment, the mice were fasted with free access to water for 8 h and then treated. Blood was collected from the orbit, and serum was obtained from the blood by centrifugation. The serum TC, TG, LDL-C and HDL-C levels were measured. A part of the liver tissue of the mice was took, homogenized, and measured for liver TC and TG contents, as well as SOD and GSH-px activities and MDA content. Another part of the liver tissue of the mice was took, fixed with 10% formaldehyde, and examined for morphology.

Experimental Results:

(1) Effect of *Saposhnikovia divaricata* polysaccharides on blood lipid level in mice with hyperlipoidemia

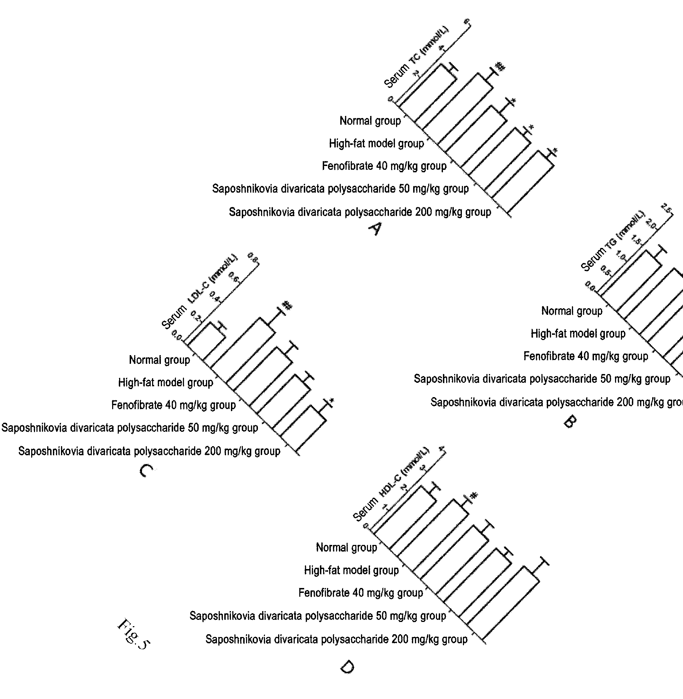

As shown in Fig. 5, the serum TC and LDL-C levels of the mice in the high-fat model group were both significantly increased (P<0.01) as compared with the normal control group. As compared with the high-fat diet group, the *Saposhnikovia divaricata* polysaccharide could lower serum TC, TG and LDL-C levels. In particular, the low dose and high dose the *Saposhnikovia divaricata* polysaccharide could significantly lower the serum TC level (P<0.05), and the high dose of the *Saposhnikovia divaricata* polysaccharide could significantly lower the serum TC, TG and LDL-C levels (P<0.05). No obvious effect on the serum HDL-C level by the *Saposhnikovia divaricata* polysaccharide was observed.

(2) Effect of *Saposhnikovia divaricata* polysaccharide on liver TC and TG contents and liver weight coefficient in mice with hyperlipoidemia

As shown in Fig. 6, the liver TC and TG contents (P<0.01) and the liver weight coefficient (P<0.01) of the mice in the high-fat model group were significantly increased as compared with the normal control group. As compared with the high-fat model group, the *Saposhnikovia divaricata* polysaccharide could lower the liver TC and TG contents and the liver weight coefficient. In particular, the low dose and high dose of the *Saposhnikovia divaricata* polysaccharide could significantly lower the liver TC content (P<0.01). As compared with the high-fat model group, the high dose of the *Saposhnikovia divaricata* polysaccharide could significantly lower the liver TC and TG contents and the liver weight coefficient (P<0.05 or P<0.01). Although the positive drug Lipanthyl could significantly lower the liver TC content (P<0.01), the liver weight coefficient was significantly increased. Therefore, the effect of the *Saposhnikovia divaricata* polysaccharide, especially a high dose of the *Saposhnikovia divaricata* polysaccharide, in lowering TC and TG, especially in lowering the liver weight coefficient, is significantly better than that of the positive drug Lipanthyl.

(3) Effect of *Saposhnikovia divaricata* polysaccharide on liver morphology of mice with hyperlipoidemia

As shown in Fig. 7, the mice in the normal control group exhibited complete liver structure and clearly visible hepatic cords, and no obvious lipid vacuole was observed (Fig. 7A). After the mice were given a high-fat diet for 3 weeks, a large number of lipid vacuoles were observed in the liver of the mice in the high-fat model group (Fig. 7B). The *Saposhnikovia divaricata* polysaccharide could significantly improve lipid vacuoles in the liver; and the high dose of the *Saposhnikovia divaricata* polysaccharide had a better effect (Figs. 7D, and 7E).

(4) Effect of *Saposhnikovia divaricata* polysaccharide on liver SOD and GSH-px activities and MDA content in mice with hyperlipoidemia

SOD and GSH-px are antioxidant enzymes in the liver, which can reduce the amount of active oxygen species and alleviate the damage of lipid peroxidation to liver cells. The experimental results are shown in Fig. 8. As compared wtih the normal control, the SOD and GSH-px activities in the liver of the mice in the high-fat model group were both significantly reduced (P<0.01), and the MDA content was significantly increased (P<0.05). After administration of the *Saposhnikovia divaricata* polysaccharide, as compared with the STZ group, the *Saposhnikovia divaricata* polysaccharide increased the liver SOD and GSH-px activities in the mice, and significantly reduced the content of MDA. In particular, both the low dose and high dose of the *Saposhnikovia divaricata* polysaccharide could significantly increase the liver SOD activity (P<0.05 or P<0.01) and the liver GSH-px activity (P<0.05 or P<0.01) in the mice, and significantly reduce the content of MDA (P<0.05).

Experimental conclusion: In mice with hyperlipoidemia induced by a high-fat diet, the *Saposhnikovia divaricata* polysaccharide can significantly reduce the serum TC, TG and LDL-C levels; meanwhile, it can also reduce the liver weight coefficient and liver TC and TG contents, and significantly reduce lipid vacuoles in the liver. Moreover, the *Saposhnikovia divaricata* polysaccharide can increase the activities of SOD and GSH-px in the liver, and reduce the content of MDA.

**Claims**

1. An isolated *Saposhnikovia divaricata* polysaccharide, comprising *L*-arabinose: *D*-galacturonic acid: *D*-mannose: *D*-glucose: *D*-galactose, wherein the molar ratio of the *L*-arabinose: *D*-galacturonic acid: *D*-mannose: *D*-glucose: *D*-galactose is 1-15 : 1-10 : 1-10 : 10-40 : 1-15, preferably 1-5 : 5-10: 1-5 : 20-25 : 1-5.

2. The isolated *Saposhnikovia divaricata* polysaccharide of claim 1, wherein,
   the *L*-arabinose includes 1,4-linked *L*-arabinose and/or 1,3,4-linked *L*-arabinose;
   the *D*-galacturonic acid includes terminal *D*-galacturonic acid;
   the *D*-mannose includes 1,6-linked *D*-mannose;
   the *D*-glucose includes 1,4-linked *D*-glucose and/or 1,3,6-linked *D*-glucose; and
   the *D*-galactose includes terminal *D*-galactose and/or 1,4-linked *D*-galactose.

3. The isolated *Saposhnikovia divaricata* polysaccharide of claim 2, wherein the molar ratio of the 1,4-linked *L*-arabinose: 1,3,4-linked *L*-arabinose: terminal *D*-galacturonic acid: 1,6-linked *D*-mannose: 1,4-linked *D*-glucose: 1,3,6-linked *D*-glucose: terminal *D*-galactose: 1,4-linked *D*-galactose is 1-5 : 1-10 : 1-10 : 1-10 : 10-30 : 1-10 : 1-5 : 1-10, preferably 1-3 : 1-5 : 5-10 : 1-5 : 15-25 : 1-5 : 1-3 : 1-5.

4. The isolated *Saposhnikovia divaricata* polysaccharide of any one of claims 1-3, wherein the isolated *Saposhnikovia divaricata* polysaccharide has a molecular weight ranging from $5 \times 10^4$ to $5 \times 10^5$ Da, preferably $1 \times 10^5$ to $3.5 \times 10^5$ Da.

5. A method for preparing the isolated *Saposhnikovia divaricata* polysaccharide of any one of claims 1-4, wherein the method comprises the steps of:

(1) extracting *Saposhnikovia divaricata* with water one or more times to give an aqueous extract of *Saposhnikovia divaricata,* optionally concentrating the aqueous extract of *Saposhnikovia divaricata*;
(2) adding an organic solvent to the optionally concentrated aqueous extract of *Saposhnikovia divaricata* to give an mixture containing the organic solvent at a concentration of 15-30%, preferably 17-28%, and more preferably 20-25%, centrifuging the mixture to give a supernatant;
(3) adding an organic solvent to the supernatant to give a mixture containing the organic solvent at a concentration of 70-90%, preferably 75-85%, and more preferably 80-85%, centrifuging the mixture to give a precipitate; and
(4) drying the precipitate to obtain the isolated *Saposhnikovia divaricata* polysaccharide.

6. The method of claim 5, wherein the volume : weight ratio of water to the *Saposhnikovia divaricata* in step (1) is 8:1 to 30:1, preferably 20:1 to 30:1.

7. The method of any one of claims 5-6, wherein the extraction temperature in step (1) is 40-100 °C, preferably 60-100 °C, and most preferably 90-95 °C.

8. The method of any one of claims 5-6, wherein the extraction time in step (1) is 1-4 hours, preferably 1-2 hours.

9. The method of any one of claims 5-6, wherein in step (1), the *Saposhnikovia divaricata* is extracted with water one or more times, preferably 1-4 times, and most preferably 2-3 times.

10. The method of claim 5, wherein the method further comprises step (3') between steps (3) and (4): dissolving the precipitate obtained from step (3) with water to give an aqueous solution, adding an organic solvent to the aqueous solution to give a mixture containing the organic solvent at a concentration of 70-90%, preferably 75-85%, and more preferably 80-85%, and centrifuging the mixture to give a precipitate; wherein step (3') can be repeated one or more times, preferably 1, 2 or 3 times.

11. The method of claim 5 or 10, wherein the organic solvent in step (2) and/or (3) and/or (3') is selected from methanol, ethanol, propanol, acetone, or a mixture thereof, preferably ethanol.

12. The method of any one of claims 5-6, wherein the *Saposhnikovia divaricata* in step (1) is a decoction piece of *Saposhnikovia divaricata.*

13. Use of the isolated *Saposhnikovia divaricata* polysaccharide of any one of claims 1-4 in the manufacture of a medicament for treating diabetes mellitus or hyperlipoidemia.

14. A pharmaceutical composition comprising the isolated *Saposhnikovia divaricata* polysaccharide of any one of claims 1-4, and a pharmaceutically acceptable carrier.

15. Use of the pharmaceutical composition of claim 14 in the manufacture of a medicament for treating diabetes mellitus or hyperlipoidemia.

16. The isolated *Saposhnikovia divaricata* polysaccharide of any one of claims 1-4 for use in the treatment of diabetes mellitus or hyperlipoidemia.

17. A method for treating diabetes mellitus or hyperlipoidemia, comprising administering to a subject in need thereof a therapeutically effective amount of the isolated *Saposhnikovia divaricata* polysaccharide of any one of claims 1-4.

Fig.1

Fig.2

Fig.3

Fig. 4

Fig. 5

Fig.6

Fig.7

Fig. 8

<div align="center">

**INTERNATIONAL SEARCH REPORT**

</div>

| International application No. |
| --- |
| **PCT/CN2019/074601** |

| A. CLASSIFICATION OF SUBJECT MATTER |
| --- |
| C08B 37/00(2006.01)i;  A61K 31/715(2006.01)i;  A61P 3/06(2006.01)i;  A61P 3/10(2006.01)i |
| According to International Patent Classification (IPC) or to both national classification and IPC |

| B. FIELDS SEARCHED |
| --- |
| Minimum documentation searched (classification system followed by classification symbols)<br>C08B; A61K; A61P |
| Documentation searched other than minimum documentation to the extent that such documents are included in the fields searched |
| Electronic data base consulted during the international search (name of data base and, where practicable, search terms used)<br>CNABS, CNKI, DWPI, SIPOABS, ISI Web of Science: 防风, 多糖, 提取物, 水提物, 沉淀, 醇沉, 甲醇, 乙醇, 丙醇, 丙酮, 酒精, 溶剂, 糖尿病, 血糖, 胰岛, 降糖, 高脂血, 高血脂, 血脂, 降脂, saposhnikovia, polysaccharide, polysaccharose, polyose, glycan, extract, precipitation, methanol, ethanol, propanol, alcohol, acetone, solvent, diabet, blood, glucose, sugar, lipid, islet, reduce, lower, decrease, hyperlipemia, hyperlipoidemia |

| C. DOCUMENTS CONSIDERED TO BE RELEVANT | | |
| --- | --- | --- |
| Category* | Citation of document, with indication, where appropriate, of the relevant passages | Relevant to claim No. |
| X | CN 1135900 A (BEIJING UNIVERSITY OF CHINESE MEDICINE) 20 November 1996 (1996-11-20)<br> description, page 1, line 7 from the bottom to page 2, line 27 | 1-4, 14, 16 |
| A | CN 1135900 A (BEIJING UNIVERSITY OF CHINESE MEDICINE) 20 November 1996 (1996-11-20)<br> entire document | 5-13, 15 |
| A | CN 107632096 A (FOSHAN UNIVERSITY) 26 January 2018 (2018-01-26)<br> entire document | 1-16 |
| A | 王松柏 (WANG, Songbai). "防风多糖的分离纯化及结构分析 (Isolation, Purification and Chemical Structure Analysis of Saposhnikovia Divaricata Polysaccharide)"<br>中国优秀硕士学位论文全文数据库医药卫生科技辑 (Medicine & Public Health, China Master Theses Full-Text Database), No. no. 11, 15 November 2006 (2006-11-15),<br> pp. 1-44 | 1-16 |

☑ Further documents are listed in the continuation of Box C.   ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
| --- | --- |
| **19 April 2019** | **07 May 2019** |

| Name and mailing address of the ISA/CN | Authorized officer |
| --- | --- |
| **National Intellectual Property Administration, PRC**<br>**No. 6, Xitucheng Road, Jimenqiao Haidian District, Beijing**<br>**100088**<br>**China** | |
| Facsimile No. **(86-10)62019451** | Telephone No. |

Form PCT/ISA/210 (second sheet) (January 2015)

**INTERNATIONAL SEARCH REPORT**

| International application No. |
| --- |
| **PCT/CN2019/074601** |

**C.      DOCUMENTS CONSIDERED TO BE RELEVANT**

| Category* | Citation of document, with indication, where appropriate, of the relevant passages | Relevant to claim No. |
| --- | --- | --- |
| A | 张泽庆 (ZHANG, Zeqing). "防风多糖的提纯, 结构分析及生物活性研究 (Extraction, Purification, Structure Analysis and Biological Activity of Polysaccharides from the Roots of saposhnikovia Devaricata)" 中国优秀硕士学位论文全文数据库工程科技I辑 (Science-Engineering (A), China Master's Theses Full-Text Database), No. no. 6, 15 June 2009 (2009-06-15), pp. 1-64 | 1-16 |
| A | 李江等 (LI, Jiang et al.). "防风多糖的研究 (Non-official translation: Study on Saposhnikovia Divaricata Polysaccharide)" 中草药 (Chinese Herbal Medicines), Vol. 30, No. (9), 15 September 1999 (1999-09-15), pp. 652-653 | 1-16 |

Form PCT/ISA/210 (second sheet) (January 2015)

**INTERNATIONAL SEARCH REPORT**

International application No.

**PCT/CN2019/074601**

| Box No. II | Observations where certain claims were found unsearchable (Continuation of item 2 of first sheet) |
|---|---|

This international search report has not been established in respect of certain claims under Article 17(2)(a) for the following reasons:

1. ☑ Claims Nos.: **17**
   because they relate to subject matter not required to be searched by this Authority, namely:

   [1]  The technical subject matter of claim 17 relates to a method for treating diabetes or hyperlipidaemia, which falls within a method for treatment of the human or animal body by therapy, and therefore does not comply with PCT Rule 39.1.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (January 2015)

**INTERNATIONAL SEARCH REPORT**
Information on patent family members

International application No.

**PCT/CN2019/074601**

| Patent document cited in search report | | | Publication date (day/month/year) | Patent family member(s) | | | Publication date (day/month/year) |
|---|---|---|---|---|---|---|---|
| CN | 1135900 | A | 20 November 1996 | CN | 1065751 | C | 16 May 2001 |
| CN | 107632096 | A | 26 January 2018 | None | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **HONGXIA DOU et al.** Research progress on the chemical components and pharmacological effects of Saposhnikovia divaricata. *Information on Traditional Chinese Medicine,* 2009, vol. 26 (2), 15
- REMINGTON'S PHARMACEUTICAL SCIENCES. Mack Publishing Company, 1995
- **ZEQING ZHANG.** Purification, structural analysis and biological activity study of Saposhnikovia divaricata polysaccharides. Master's Thesis of Shaanxi Normal University, 2008, 77
- **LIN GAO.** Quantitative Determination of Uronic Acid in MCP. *Chemical Industry and Engineering,* 2005, vol. 22 (6), 487-489
- **JIE ZHANG.** Determination of the basic content of Phellodendron amurense polysaccharides before and after stir-heating with a salt solution and its effect on immune function. *Liaoning Journal of Traditional Chinese Medicine,* 2017, vol. 44 (6), 1263-1267
- **QIAN CHEN.** Determination of the sulfate content in fucoidan by barium sulfate-turbidimetry. *Journal of Pharmaceutical Practice,* 2012, vol. 30 (2), 118-120
- **HOUQIANG DING.** Determination of molecular weight and distribution of hyaluronic acid by combination of multi-angle laser light scattering spectrometer and size exclusion chromatography. *Food and Drug,* 2009, vol. 11 (3), 24-26
- **JINIAN FANG.** *Methylation analysis methods of polysaccharides, Foreign Medical Sciences (Section of Pharmacology),* 1986, vol. 4, 222-226
- **LIYAN SUN ; ZHENLIANG LIU ; JINXIA SUN et al.** Effect of Imperata cylindrica polysaccharide on hypoxia tolerance in mice. *China Journal of Hospital Pharmacy,* 2008, vol. 28 (2), 96-99
- Intervention of immunoregulation and renal fibrosis. **BIN LENG.** Imperata cylindrica polysaccharide in rats with IgA nephropathy. Dissertations of Guilin Medical University, 2013
- **SHIJING LV ; QICAI LONG ; DEYUAN HE et al.** Regulation of lymphocyte proliferation and T cell subpopulation by Imperata cylindrica polysaccharide in patients with hepatitis B. *Conference Paper] 2001 - The Second National Academic Conference on Immunology of Traditional Chinese Medicine*